# EUROPEAN PATENT APPLICATION

(11) **EP 1 454 644 A1**
(43) Date of publication of application: **08.09.2004**
(21) Application number: 04004679.9
(22) Date of filing: 01.03.2004
(51) Int. Cl.: A61M 5/142

(54) **Device for transferring a fluid**

(30) Priority: 04.03.2003 IT MO20030056
(71) Applicant: Sidam di Azzolini Graziano E C. S.a.s., 41030 San Giacomo Roncole - Mirandola, (Prov. of Modena) (IT)
(72) Inventor: Azzolini, Graziano, 41032 Cavezzo (Prov. of Modena) (IT)
(74) Representative: Modiano, Guido, Dr.-Ing.

(57) **Abstract**

A device for transferring a fluid contained in a closed container (2) comprising a substantially tubular connecting element (7) that has a first end (8) that can be inserted hermetically in the container (2) and a second end (9) that is open, and pumping elements (10) that have an inlet (11), which can be associated hermetically with the second end (9), and an outlet (12), and are adapted to draw the fluid from the container (2) in order to transfer it into an external user device (U); the transfer device (1) is assembled monolithically.

## Description

The present invention relates to a device for transferring a fluid.

In the medical and surgical field in general, many operations and examinations must be performed in a sterile environment in order to protect the patients against possible biological contamination, particularly if the patients are in an altered immune state.

A sterile environment is substantially constituted by a room that is furnished at least with a table on which the patient lies and with a trolley on which the instruments for performing the operation or examination are placed; the room, the table, the trolley and of course the instruments are sterile.

It is also known that performing certain operations or examinations requires the use of fluids, such as for example physiological solutions, contrast media, medicines or others.

These fluids are generally packaged in hermetically sealed containers that are suitable to keep them intact and free from contamination until they are used; the containers can be constituted for example by bottles closed by a rubber stopper covered with a protective cap that is rigidly coupled to the bottle by a tear-off locking ring.

As an alternative, the containers can be constituted by a closed pouch provided with an access nozzle, which is also closed hermetically by a stopper or the like.

The outer surface of these containers is not sterile, and therefore said containers cannot be placed proximate to the table where the patient lies or on the worktop of the instrument supporting trolley, where perfect sterility must be guaranteed: accordingly, the containers must be kept at a distance that limits the risks of contamination of the patient and of the instruments.

For these reasons, the sanitary personnel working in the sterile room, after opening the containers, transfers the doses of fluids required in each instance into sterile trays of the open-top type, which are arranged on the instrument trolley, and then the open containers are moved away.

The transfer is performed by pouring, by gravity, tipping the container so as to make the fluid flow out through the opening formed in said container.

These operations are very awkward, time-consuming and unsafe for the assigned personnel and for the patients.

In fact, the containers are opened, by tearing the locking rings and removing the corresponding stoppers, with the aid of pliers, with which the personnel may accidentally tear the gloves they are wearing and their skin, thus leaving open paths of possible contamination.

During pouring there can be accidental spillage of the fluid outside the trays; moreover, significant and unused amounts of fluid remain inside the containers, accordingly entailing an onerous waste of materials.

Finally, while the open containers are idle between two successive transfers, the fluids contained therein can be contaminated, with consequent disadvantageous risk of patient infection.

The aim of the present invention is to eliminate the above-noted drawbacks, by providing a device for transferring a fluid that allows to draw the fluid from the corresponding container simply, rapidly, conveniently and safely, to ensure the sterility of the fluid between successive withdrawals, and to limit the waste of materials.

Within this aim, an object of the present invention is to provide a device for transferring a fluid that is simple, relatively easy to provide in practice, safe in use, effective in operation, and has a relatively low cost.

Another object of the present invention is to ensure the safety of the sanitary personnel and of the patients.

This aim and these and other objects that will become better apparent hereinafter are achieved by the present device for transferring a fluid contained in a closed container, characterized in that it comprises a substantially tubular connecting element that has a first end that can be inserted hermetically in said container and a second end that is open, and pumping means that have an inlet, which can be associated hermetically with said second end, and an outlet, and are suitable to draw said fluid from said container in order to transfer it into an external user device.

Further characteristics and advantages of the present invention will become better apparent from the following detailed description of a preferred but not exclusive embodiment of a device for transferring a fluid, illustrated by way of non-limiting example in the accompanying drawings, wherein:

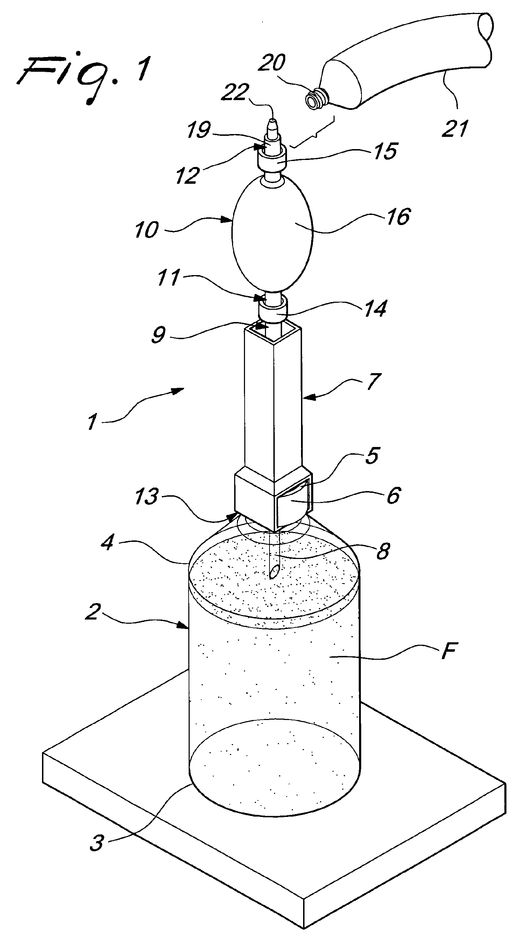

Figure 1 is a schematic axonometric view of a device for transferring a fluid according to the invention, applied to a container;
Figure 2 is a schematic axonometric view of a device according to the invention in the transfer configuration.

With reference to the figures, the reference numeral 1 generally designates a device for transferring a fluid F.

The fluid F is contained in a container 2, such as for example a bottle 3 provided with a neck 4 at the top of which an opening is provided that is closed hermetically by a closure element made of a pierceable material, such as for example a rubber stopper covered with a cap 5 made of metal or plastics and provided with a ring 6 for locking to the neck 4.

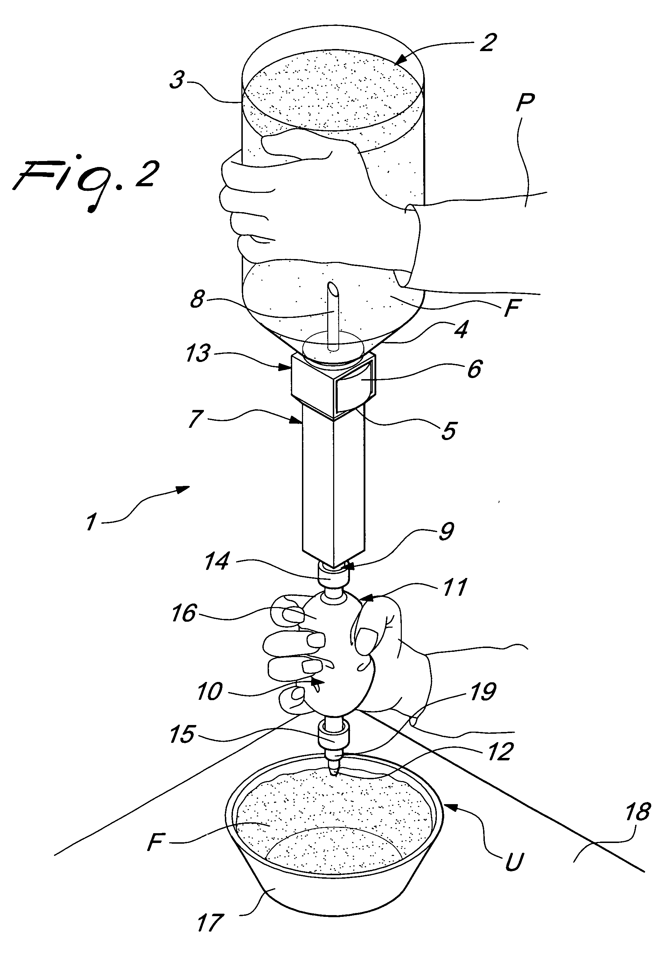

In an alternative embodiment, of a conventional type, the container 2 can be constituted by a pouch, a bag or the like, that has, for example, a shape such as that of the bottle 3 shown in Figure 2 and is made of flexible or semirigid material, for example plastic material, which is closed along all its sides and is provided with a nozzle in which there is an access opening that is also closed hermetically by a closure element made of pierceable material.

The device 1 comprises a substantially tubular connecting element 7, which is provided with a first end, which can be inserted hermetically in the container 2 and is constituted for example by a cannula 8 for piercing the rubber stopper or other closure element with which the container 2 is provided, and with a second end 9, which is open.

The device 1 further comprises pumping means 10, which are provided with an inlet 11, which can be associated hermetically with the second end 9 of the connecting element 7, and with an outlet 12; the pumping means 10 are suitable to draw the fluid F from the container 2 to transfer it into an external user device U.

The cannula 8 pierces the rubber stopper that closes the container 2 and extends within said container.

Moreover, the device 1 is provided with means 13 for fixing to the outside of the container 2; the fixing means 13, for example of the engagement type or of another conventionally known type, can be formed in the connecting element 7 proximate to the cannula 8 and ensure a stable connection of the connecting element 7, and therefore of the entire device 1, which is assembled monolithically, to the container 2.

First valve means 14 are interposed between the second end 9 of the connecting element 7 and the inlet 11 of the pumping means 10, and second valve means 15 are associated with the outlet 12 of the pumping means 10.

The first valve means 14 and the second valve means 15, shown schematically in the figures because they are of a conventional type, are of the unidirectional type in the direction of flow for transferring the fluid F from the container 2 to the user device U.

The first valve means 14 can be for example of the type with a floating ball or the like and are suitable to allow the fluid F to flow from the container 2 to the pumping means 10 but not the reverse; the second valve means 15 can instead be for example of the bevel-cut type or the like and are suitable to allow the flow of the fluid out of the outlet 12 but not the reverse.

The pumping means 10 can be constituted by a pump 16 made of elastically deformable material that can be actuated manually: the compression and subsequent release of the pump 16, together with the flow control determined by the first and second valve means 14 and 15, allow to draw the fluid F from the container 2 and transfer it through the outlet 12 toward the user device U.

The user device U to which the fluid F drawn from the container 2 is fed can be constituted by an open-top sterile tray 17 that is arranged on the worktop 18 of an instrument trolley of the type normally used in sterile rooms.

As an alternative, the user device U to which the fluid F drawn from the container 2 is fed can be constituted by the inlet tube of a circuit or a medical apparatus that is connected directly to the patient and is not shown.

Conveniently, at the outlet 12 a connection element 19 is provided for connection to another complementary connecting element 20 that is associated with a duct 21 for feeding to the inlet tube of a circuit or a medical apparatus connected to the patient.

The connecting element 19 and the complementary connecting element 20 can be for example of the Luer-Lock male and female type, or vice versa, although alternative embodiments are not excluded.

The device 1 further comprises a closure element that is constituted for example by a stopper 22 that is detachably associable with the outlet 12 so as to preserve the integrity and sterility of the fluid F between successive withdrawals, particularly if the outlet 12 is not directly and permanently connected, for example by means of the duct 21, to a circuit or a medical apparatus and the transfer is performed discontinuously inside the tray 17.

Advantageously, the device 1 can be provided with tubular extensions, which can be similar to the tube 7 and which have an end that is associable with the outlet 12.

With particular reference to the embodiment shown in Figure 2, the operation of the invention is as follows.

A member of personnel P, after taking hold of the container 2 that contains the fluid F to be used and is still hermetically closed, and after taking hold of the device 1, which is already assembled monolithically, forces the cannula 8 to penetrate through the rubber stopper that closes the container 2 without having to first eliminate the ring 6 or the stopper, and anchors the device 1 to the container 2 by way of the fixing means 13.

When it is necessary to transfer a chosen amount of fluid F into the tray 17, the member of personnel P overturns the container 2 to which the device 1 has been applied, and after removing the stopper 22 acts manually on the pumping means 10 with successive compressions and decompressions on the pump 16, as a consequence of which the fluid F is aspirated from the container 2 to flow out into the tray 17 thanks to the automatic intervention of the first and second valve means 14 and 15.

Once the transfer has occurred, the member of personnel P reapplies the stopper 22 to the outlet 12 in order to isolate the residual fluid F from the outside environment and thus preserve its integrity and sterility until the subsequent withdrawal.

In practice it has been found that the described invention achieves the intended aim and objects.

The invention thus conceived is susceptible of numerous modifications and variations, all of which are within the scope of the appended claims.

All the details may further be replaced with other technically equivalent ones.

In practice, the materials used, as well as the shapes and dimensions, may be any according to requirements without thereby abandoning the scope of the protection of the appended claims.

The disclosures in Italian Patent Application No. M02003A000056 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A device for transferring a fluid contained in a closed container (2), **characterized in that** it comprises a substantially tubular connecting element (7) that has a first end (8) that can be inserted hermetically in said container (2) and a second end (9) that is open, and pumping means (10) that have an inlet (11), which can be associated hermetically with said second end (9), and an outlet (12), and are suitable to draw said fluid from said container (2) in order to transfer the fluid into an external user device (U).

2. The device according to claim 1, **characterized in that** it comprises first valve means (4) interposed between said second end (9) and said inlet (11).

3. The device according to one or more of the preceding claims, **characterized in that** it comprises second valve means (15) associated with said outlet (12).

4. The device according to one or more of the preceding claims, **characterized in that** said first valve means (14) and/or said second valve means (15) are of the unidirectional type in the direction of the flow for transferring the fluid from said container (2) to said user device (U).

5. The device according to one or more of the preceding claims, **characterized in that** said first valve means (14) are of the type with a floating ball or the like and are suitable to allow the fluid to flow from said container (2) to said pumping means (10).

6. The device according to one or more of the preceding claims, **characterized in that** said second valve means (15) are of the bevel-cut type or the like and are suitable to allow the fluid to flow out of said outlet (12).

7. The device according to one or more of the preceding claims, **characterized in that** said outlet (12) is provided with an element (19) for connection to another complementary element (20) associated with said user device (U).

8. The device according to one or more of the preceding claims,
**characterized in that** said connecting element (19) is of the Luer-Lock male or female type or the like.

9. The device according to one or more of the preceding claims, **characterized in that** it comprises a closure element (22) that is detachably associable with said outlet (12).

10. The device according to claim 9, **characterized in that** said closure element is a stopper (22) or the like.

11. The device according to one or more of the preceding claims, **characterized in that** it comprises tubular extensions provided with an end that is associable with said outlet (12) of said pumping means (10).

12. The device according to one or more of the preceding claims, **characterized in that** said first end of the connecting element (7) is constituted by a piercing cannula (8).

13. The device according to one or more of the preceding claims, **characterized in that** it comprises means (13) for fixing to said container (2).

14. The device according to one or more of the preceding claims, **characterized in that** said fixing means (13) are formed in said connecting element (7) and are of the engagement type or the like.

15. The device according to one or more of the preceding claims, **characterized in that** it is provided assembled monolithically.

16. The device according to one or more of the preceding claims, **characterized in that** said container (2) is a bottle or the like and is closed hermetically by a closure element (5) made of a pierceable material.

17. The device according to one or more of the preceding claims, **characterized in that** said container (2) is constituted by a pouch or the like that is closed and is provided with a nozzle in which there is an opening that is closed hermetically by a closure element (5) made of a pierceable material.

18. The device according to one or more of the preceding claims, **characterized in that** said pumping means (10) are of the type of a pump made of elastically deformable material that can be operated manually, the compression and subsequent release of said pump (10) being suitable to draw the fluid from said container (2) and transfer it through said outlet (12) toward said user device (U).

19. The device according to one or more of the preceding claims, **characterized in that** said user device (U) is an open-top tray or the like.

20. The device according to one or more of the preceding claims, **characterized in that** said user device is a tube or the like for input to a circuit or to a medical apparatus or the like that uses said fluid.
